# EUROPEAN PATENT SPECIFICATION

(11) **EP 1 200 826 B1**
(45) Date of publication and mention of the grant of the patent: **23.06.2004**
(21) Application number: 00947920.5
(22) Date of filing: 03.07.2000
(51) Int. Cl.: G01N 33/543

(54) **INTERNALLY REFERENCED IMMUNOASSAY AND TEST DEVICE**
IMMUNOASSAY UND TEST-VORRICHTUNG MIT INTEGRIERTER REFERENZ
TEST ET IMMUNODOSAGE A REFERENCE INTERNE

(30) Priority: 11.08.1999 EP 99306347
(43) Date of publication of application: 02.05.2002
(73) Proprietor: UNILEVER PLC, London EC4P 4BQ (GB); UNILEVER N.V., 3013 AL Rotterdam (NL)
(72) Inventor: BADLEY, Robert Andrew, Unilever Research Colworth, Bedford, Bedfordshire MK44 1LQ (GB); HOWELL, Steven, Unilever Research Colworth, Bedford, Bedfordshire MK44 1LQ (GB); VAN DER LOGT, C.P.E., Unilever Researh Vlaardingen, NL-3133 AT Vlaardingen (NL)
(74) Representative: Lipscombe, Martin John
(86) International application number: PCT/EP2000/006235
(87) International publication number: WO 2001/013116

(56) References cited:
- EP-A- 0 034 050
- EP-A- 0 041 426
- EP-A- 0 093 613
- EP-A- 0 274 198
- EP-A- 0 833 157
- US-A- 5 869 620

### SEQUENCE LISTING

<110> UNILEVER PLC
<120> INTERNALLY REFERENCED IMMUNOASSAY AND TEST DEVICE
<130> P3107(C)
<140> 99306347.8-2204
<141> 1999-08-11
<160> 8
<170> PatentIn Ver. 2.1
<210> 1
<211> 500
<212> PRT
<213> Artificial Sequence
<220>
<223> Description of Artificial Sequence:expression product
<400> 1
   His His His His His His Gly Ser Tyr Gly Ile His Arg Pro Val Ala
     1               5                  10                  15
   Thr Met Val Ser Lys Gly Glu Glu Leu Phe Thr Gly Val Val Pro Ile
                20                  25                  30
   Leu Val Glu Leu Asp Gly Asp Val Asn Gly His Lys Phe Ser Val Ser
            35                  40                  45
   Gly Glu Gly Glu Gly Asp Ala Thr Tyr Gly Lys Leu Thr Leu Lys Phe
        50                  55                  60
   Ile Cys Thr Thr Gly Lys Leu Pro Val Pro Trp Pro Thr Leu Val Thr
    65                  70                  75                  80
   Thr Leu Thr Tyr Gly Val Gln Cys Phe Ser Arg Tyr Pro Asp His Met
                    85                  90                  95
   Lys Gln His Asp Phe Phe Lys Ser Ala Met Pro Glu Gly Tyr Val Gln
               100                 105                 110
   Glu Arg Thr Ile Phe Phe Lys Asp Asp Gly Asn Tyr Lys Thr Arg Ala
           115                 120                 125
   Glu Val Lys Phe Glu Gly Asp Thr Leu Val Asn Arg Ile Glu Leu Lys
       130                 135                 140
   Gly Ile Asp Phe Lys Glu Asp Gly Asn Ile Leu Gly His Lys Leu Glu
   145                 150                 155                 160
   Tyr Asn Tyr Asn Ser His Asn Val Tyr Ile Met Ala Asp Lys Gln Lys
   165 170 175
   Asn Gly Ile Lys Val Asn Phe Lys Ile Arg His Asn Ile Glu Asp Gly
   180 185 190
   Ser Val Gln Leu Ala Asp His Tyr Gln Gln Asn Thr Pro Ile Gly Asp
   195 200 205
   Gly Pro Val Leu Leu Pro Asp Asn His Tyr Leu Ser Thr Gln Ser Ala
   210 215 220
   Leu Ser Lys Asp Pro Asn Glu Lys Arg Asp His Met Val Leu Leu Glu
   225 230 235 240
   Phe Val Thr Ala Ala Gly Ile Thr Gly Gly Ser Gly Gly Gly Gly Ser
   245 250 255
   Ser Gln Val Lys Leu Leu Glu Ser Gly Gly Glu Leu Val Gln Pro Gly
   260 265 270
   Gly Ser Leu Lys Leu Ser Cys Ala Ala Ser Gly Leu Thr Phe Thr Asn
   275 280 285
   Tyr Ser Met Gly Trp Phe Arg Pro Gly Pro Gly Val Asp Arg Glu Ala
   290 295 300
   Val Ala Ala Ile Ser Trp Ser Gly Asp Asn Thr Tyr Tyr Val Ser Ser
   305 310 315 320
   Val Lys Gly Arg Phe Thr Ile Ser Arg Asp Asn Ala Lys Asn Thr Val
   325 330 335
   Tyr Leu Gln Met Asn Ser Leu Lys Pro Gln Asp Thr Ala Val Tyr Tyr
   340 345 350
   Cys Ala Val Lys Pro Asp Asp Gly Trp Trp Asp Tyr Trp Gly Gln Phe
   355 360 365
   Thr Gln Val Thr Val Ser Ser Gln Val Gln Leu Gln Glu Ser Gly Gly
   370 375 380
   Gly Leu Val Gln Ala Gly Glu Ser Leu Lys Leu Ser Cys Ala Ala Ser
   385 390 395 400
   Gly Asn Thr Phe Ser Gly Gly Phe Met Gly Trp Tyr Arg Gln Ala Pro
   405 410 415
   Gly Lys Gln Arg Glu Leu Val Ala Thr Ile Asn Ser Arg Gly Ile Thr
   420 425 430
   Asn Tyr Ala Asp Phe Val Lys Gly Arg Phe Thr Ile Ser Arg Asp Asn
   435 440 445
   Ala Lys Lys Thr Val Tyr Leu Glu Met Asn Ser Leu Glu Pro Glu Asp
   450 455 460
   Thr Ala Val Tyr Tyr Cys Tyr Thr His Tyr Phe Arg Ser Tyr Trp Gly
   465 470 475 480
   Gln Gly Thr Gln Val Thr Val Ser Ser Glu Gln Lys Leu Ile Ser Glu
   485 490 495
   Glu Asp Leu Asn
   500
<210> 2
<211> 495
<212> PRT
<213> Artificial Sequence
<220>
<223> Description of Artificial Sequence:expression product
<400> 2
   Met Lys Tyr Leu Leu Pro Thr Ala Ala Ala Gly Leu Leu Leu Leu Ala
   1 5 10 15
   Ala Gln Pro Ala Met Ala Gln Val Gln Leu Gln Gln Ser Gly Ala Glu
   20 25 30
   Leu Val Lys Pro Gly Pro Ser Val Lys Leu Ser Cys Lys Ala Ser Asp
   35 40 45
   Tyr Thr Phe Thr Ser Tyr Trp Met His Trp Val Lys Gln Arg Pro Gly
   50 55 60
   Gln Gly Leu Glu Trp Ile Gly Glu Ile Asn Pro Thr Asn Gly Arg Thr
   65 70 75 80
   Tyr Tyr Asn Glu Lys Phe Lys Ser Lys Ala Thr Leu Thr Val Asp Lys
   85 90 95
   Ser Ser Ser Thr Ala Tyr Met Gln Leu Ser Ser Leu Thr Ser Glu Asp
   100 105 110
   Ser Ala Val Tyr Tyr Cys Ala Arg Arg Tyr Gly Asn Ser Phe Asp Tyr
   115 120 125
   Trp Gly Gln Gly Thr Thr Val Thr Val Ser Ser Gln Val Gln Leu Gln
   130 135 140
   Gln Ser Gly Pro Glu Leu Val Lys Pro Gly Ala Ser Val Lys Met Ser
   145 150 155 160
   Cys Lys Ala Ser Gly Tyr Thr Phe Thr Ser Tyr Val Met His Trp Val
   165 170 175
   Lys Gln Lys Pro Gly Gln Gly Leu Glu Trp Ile Gly Tyr Ile Tyr Pro
   180 185 190
   Tyr Asn Asp Gly Thr Lys Tyr Asn Glu Lys Phe Lys Gly Lys Ala Thr
   195 200 205
   Leu Thr Ser Asp Lys Ser Ser Ser Thr Ala Tyr Met Glu Leu Ser Ser
       210                 215                 220
   Leu Thr Ser Glu Asp Ser Ala Val Tyr Tyr Cys Ser Arg Arg Phe Asp
   225                 230                 235                 240
   Tyr Trp Gly Gln Gly Thr Thr Val Thr Val Ser Ser Met Lys Tyr Leu
                   245                 250                 255
   Leu Pro Thr Ala Ala Ala Gly Leu Leu Leu Leu Ala Ala Gln Pro Ala
               260                 265                 270
   Met Ala Asp Ile Glu Leu Thr Gln Ser Pro Ser Ser Met Tyr Ala Ser
           275                 280                 285
   Leu Gly Glu Arg Ile Thr Ile Thr Cys Lys Ala Ser Gln Asp Ile Asn
       290                 295                 300
   Thr Tyr Leu Thr Trp Phe Gln Gln Lys Pro Gly Lys Ser Pro Lys Thr
   305                 310                 315                 320
   Leu Ile Tyr Arg Ala Asn Arg Leu Leu Asp Gly Val Pro Ser Arg Phe
                   325                 330                 335
   Ser Gly Ser Gly Ser Gly Gln Asp Tyr Ser Leu Thr Ile Ser Ser Leu
               340                 345                 350
   Asp Tyr Glu Asp Met Gly Ile Tyr Tyr Cys Leu Gln Tyr Asp Glu Leu
           355                 360                 365
   Tyr Thr Phe Gly Gly Gly Thr Lys Leu Glu Ile Lys Arg Asp Ile Glu
       370                 375                 380
   Leu Thr Gln Ser Pro Asp Ser Leu Ala Val Ser Leu Gly Gln Arg Ala
   385                 390                 395                 400
   Thr Ile Ser Cys Arg Ala Ser Glu Ser Val Asp Ser Tyr Gly Asn Ser
                   405                 410                 415
   Phe Met Gln Trp Tyr Gln Gln Lys Pro Gly Gln Pro Pro Lys Leu Leu
               420                 425                 430
   Ile Tyr Arg Ala Ser Asn Leu Glu Ser Gly Ile Pro Ala Arg Phe Ser
           435                 440                 445
   Gly Thr Gly Ser Arg Thr Asp Phe Thr Leu Thr Ile Asn Pro Val Glu
       450                 455                 460
   Ala Asp Asp Val Ala Thr Tyr Tyr Cys Gln Gln Ser Asp Glu Tyr Pro
   465                 470                 475                 480
   Tyr Met Tyr Thr Phe Gly Gly Gly Thr Lys Leu Glu Ile Lys Arg
                   485                 490                 495
<210> 3
<211> 47
<212> DNA
<213> Artificial Sequence
<220>
<223> Description of Artificial Sequence:synthentic insert
<220>
<221> CDS
<222> (3)..(44)
<400> 3
   ct ctc gag aaa aga cat cac cat cac cat cac ggc tct tac gta cag     47
      Leu Glu Lys Arg His His His His His His Gly Ser Tyr Val
        1               5                  10
<210> 4
<211> 13
<212> PRT
<213> Artificial Sequence
<223> Description of Artificial Sequence:synthentic insert
<400> 4
   Leu Glu Lys Arg His His His His His His Gly Ser Tyr
     1               5                  10
<210> 5
<211> 38
<212> DNA
<213> Artificial'Sequence
<220>
<223> Description of Artificial Sequence: synthetic oligonucleotide
<400> 5
   tcgagaaaag acatcaccat caccatcacg gctcttac                          38
<210> 6
<211> 34
<212> DNA
<213> Artificial Sequence
<220>
<223> Description of Artificial Sequence:synthetic oligonucleotide
<400> 6
   gtaagagccg tgatggtgat ggtgatgtct tttc                              34
<210> 7
<211> 21
<212> DNA
<213> Artificial Sequence
<220>
<223> Description of Artificial Sequence:linker
<400> 7
   gactggttcc aattgacaag c 21
<210> 8
<211> 60
<212> DNA
<213> Artificial Sequence
<220>
<223> Description of Artificial Sequence:linker
<400> 8
   atcgaattct ctagatccac cgcctccaga accgccagtg atcccggcgg cggtcacgaa 60

## Description

### Field of the Invention

This invention relates to immunoassays involving specific binding, and to test devices for carrying out such assays. In particular, this invention relates to immunoassays (and test devices) exhibiting improved reproducibility as a result of the assay containing an internal reference, the internal reference enabling calibration of the assay result to account for fluctuations in the amount of assay binding reagents.

### Background of the Invention

Immunoassays which make use of the specific binding properties of antibody-type molecules to detect the presence of certain analytes in a sample are well known in the art. Essentially, such immunoassays function by bringing a sample into contact with various binding reagents so as to enable the formation of a complex consisting of the analyte and one or more antibody-type molecules, and then subsequently detecting the presence or absence of the complex on a surface or in a solution.

Immunoassays can take any one of a number of specific forms, depending on the type of analyte they are designed to measure and/or the particular diagnostic setting in which they are used. For example, immunoassays can be either competitive or non-competitive (i.e: "sandwich-type") in nature, both of which have been described in the literature in considerable detail. Additionally, immunoassays may incorporate a variety of binding reagents, and such variety, both in the number and type of binding reagents used, can provide a means by which to modulate an assay's selectivity and sensitivity to a variety of analytes. Immunoassays may also be constructed in such a way as to provide a variety of types of assay signals, for example, signals which are visible to the human eye, or signals which are measurable only with the assistance of complex monitoring equipment.

As the foregoing implies, modern commercially utilised immunoassays are complex diagnostic tools. As such, there is significant potential for variability to exist among different tests for the same analyte, even when using the same assay device. Most assays are at least in part sensitive to temperature, development time, incubation, sample size and reagent stability, each of which, if varied, could give rise to variable, and hence unacceptable, results. Another potential source of error in immunoassays is the variability in the amount of binding reagent to which a given sample is exposed. Because of this, it is desirable to internally reference the level of such reagents, and to correct for any variation in their level among successive assay tests.

In this regard, Attridge et al. WO 92/09892, Gunars et al. AU-A-70447/87 and Litman et al. EP-A-0093613 all describe immunoassays incorporating a form of internal referencing capable of correcting for variability in test results. EP-A-0093613, in particular, describes an immunoassay (and test device) which utilises a measurement surface and a calibration surface. The measurement surface involves the binding of a conjugate to the surface by means of specific binding pair complex formation, where the conjugate comprises two distinct components, namely a member of a signal producing system bound to a member of a specific binding pair. The calibration surface, by contrast, involves a signal producing member bound to the surface, either initially or through the intermediacy of specific binding pair complex formation, where the specific binding pair is different from the binding pair of the measurement surface.

Although EP-A-0093613 provides for internal referencing, it does so in a manner which has apparent disadvantages. EP-A-0093613 requires, in either competitive or non-competitive assay formats, the construction of a multitude of different specific binding pair members, each of which is independently susceptible to inactivation by denaturing if temperature or other testing conditions vary. Such inactivation clearly is undesired as it can lead to unacceptable variability in test results.

Internally referenced immunoassays as described in WO 92/09892 and AU-A-70447/87 likewise exhibit these same deficiencies.

### Summary of the Invention

It would therefore be desirable to provide an immunoassay capable of overcoming the foregoing disadvantages, and to provide an analytical test device capable of performing such an immunoassay. In this regard, the present invention provides a method of detecting the presence of an analyte in a sample, the method comprising:
a) contacting the sample with a binding reagent and an analyte receptor to form a first complex comprising the binding reagent, analyte, and analyte receptor, the first complex providing a test signal, wherein the analyte receptor is located within a test zone and is capable of binding to the analyte, and the binding reagent has a binding specificity for the analyte;
b) contacting the sample with a reference ligand to form a second complex comprising the binding reagent and reference ligand, the second complex providing a reference signal, wherein the reference ligand is located within a reference zone, and further wherein the binding reagent has a binding specificity for the reference ligand; and
c) detecting from the test and reference zones the test and reference signals, whereby the ratio of the test signal to the reference signal, when taken, defines the level of analyte in the sample, such level being independent of the level of binding reagent contacted with the sample.

Also contemplated is an analytical test device for detecting the presence of an analyte in a liquid biological sample by specific binding of the analyte to a binding reagent to which the sample is exposed, and an analyte receptor, to form a first complex, wherein the binding reagent is selected from an antibody or a multivalent antigen binding protein, the antibody and multivalent antigen binding protein each comprising a first and second domain binding unit, wherein the first domain binding unit provides a binding specificity for the analyte and the second domain binding unit provides a binding specificity for a reference ligand; the device comprising a first and second solid support, the first solid support comprising a reference zone, the reference zone having irreversibly immobilised thereon the reference ligand which, when bound to the binding reagent, forms a second complex which provides a reference signal which is a means by which to correct for variations in the amount of binding reagent exposed to the sample; and the second solid support comprising a test zone, the test zone having irreversibly immobilised thereon the analyte receptor which, when bound to the analyte and binding reagent, forms the first complex which provides a test signal.

The immunoassays of the invention provide for assay devices to be constructed which are capable of accurate and reproducible test results, ideally suited for commercial markets such as the clinical or home-testing markets. The immunoassays are internally referenced by use of a binding reagent specifically constructed to avoid the problems inherent in the prior art. As the binding reagent comprises on a single molecule the two domain binding units necessary for analyte measurement and internal referencing, there are fewer opportunities for the denaturing of assay components under variable test conditions to occur, and hence variability of test results is minimised. Furthermore, as the binding reagent utilised in the present invention is but a single molecule rather than a complex of two or more molecules, if denaturing does occur, the likelihood is that the entire molecule will denature, thus providing that the signal ratio of test to reference signals will be unaffected.

### Brief Description of the Figures

Figure 1 shows the amino acid sequence of SEQ ID NO:1, a llama bi-head green fluorescent protein binding reagent which contains two binding sites, one for human chorionic gonadotrophin (hCG) and one for reactive red 6 dye (RR6).
Figure 2 shows the amino acid sequence of SEQ ID NO:2, a double-head antibody binding reagent which contains two binding sites, one for human chorionic gonadotrophin (hCG) and one for glucose oxidase (GOx).

### Detailed Description of the Invention

The immunoassays of the invention may be used to determine the presence of any number of known analytes. Representative analytes include drugs, metabolic analytes (e.g. enzymes), proteins, nucleic acids or carbohydrates. Alternatively, the analyte may be of a macromolecular or particulate nature, such as allergens (e.g. dust mite faeces), bacteria (e.g. *chlamydia* or *salmonella*) or virus particles or components thereof, or other micro-organisms such as fungi or bacterial spores. Of particular interest are hormonal analytes, especially sex and/or fertility hormones and analogues thereof, such as estrone-3-glucuronide (E3G), pregnanediol-3-glucuronide (P3G), human chorionic gonadotrophin (hCG), lutenizing hormone (LH) and follicle stimulating hormone (FSH).

The immunoassays may be applied to virtually any type of biological or non-biological sample, though liquid biological samples derived from urine, serum, tears, saliva, cervical, tears or urethral fluids, and stool samples are most typically used. Other samples which are suitable for the assays of the invention include liquid or solid food samples and samples from industrial environments. The samples may be purified or diluted prior to assaying.

If samples are used which contain bacterial or viral analytes, it may be necessary to first contact the sample with an extraction buffer capable of extracting the analyte of interest from the bacteria or virus. Extraction may be performed by any known means, such as through the use of zwitterionic detergents or non-ionic detergents.

Once a suitable sample has been identified, the immunoassays of the invention can be used to identify the presence or absence of a particular analyte in the sample. This will occur by
a) contacting the sample with a binding reagent and an analyte receptor to form a first complex comprising the binding reagent, analyte, and analyte receptor, the first complex providing a test signal, wherein the analyte receptor is located within a test zone and is capable of binding to the analyte, and the binding reagent has a binding specificity for the analyte;
b) contacting the sample with a reference ligand to form a second complex comprising the binding reagent and reference ligand, the second complex providing a reference signal, wherein the reference ligand is located within a reference zone, and further wherein the binding reagent has a binding specificity for the reference ligand; and
c) detecting from the test and reference zones the test and reference signals, whereby the ratio of the test signal to the reference signal, when taken, defines the level of analyte in the sample, such level being independent of the level of binding reagent contacted with the sample.

The binding reagent in this assay may be any compound having two distinct binding specificities, one of which is for the analyte of interest and the other is for a reference ligand. By binding specificity, it is meant that the binding reagent is capable of binding to an epitope of interest in a selective fashion in the presence of excess quantities of other materials not of interest, and tightly enough (with high enough affinity) to provide a useful assay. Examples of standard reagents which exhibit specific binding include antibodies which bind only to one species of bacteria but not to other species. By "distinct", it is meant that the binding reagent exhibits two different binding specificities to two different molecules, or to two different sites on the same molecule, such that steric or other interference between the resulting two binding events is negligible and does not significantly affect the ability of the binding reagent to bind both molecules or sites concurrently.

In a preferred embodiment of the invention, the binding reagent is selected from an antibody or a multivalent antigen binding protein, the antibody and multivalent antigen binding protein each comprising a first and second domain binding unit, wherein the first domain binding unit provides a binding specificity for the analyte and the second domain binding unit provides a binding specificity for the reference ligand. A domain binding unit, as used herein, means an immunoglobulin variable domain which naturally forms a complete antigen binding site.

Various forms of antibodies are contemplated which may include such antibody-type molecules and constructs as Fv, Fab, ScFv and the like. Specific examples of antibodies suitable for the present invention include those multivalent and/or multispecific constructions which have been described in the literature and comprise two or more polypeptide chains -- see for example, patent application Harris et al., WO 94/09131 and Davis et al., WO 97/14719 -- or are based on a 'double ScFv' approach, wherein the multivalency arises when two or more monovalent ScFv molecules are linked together, providing a single chain molecule comprising at least four variable domains, as described, for example, in Whitlow et al., WO 93/11161 and Mezes et al., WO 94/13806. In all of these cases, the domain binding units are formed through the association of light and heavy chain variable domains. Other suitable binding reagents may be derived from such antibodies as those described in Casterman et al., EP-A-0584421, which discloses fragments of heavy chain immunoglobulins devoid of light chains, including fragments corresponding to isolated V_{H} domains or to V_{H} dimers linked by the hinge disulphide.

The binding reagent of the present invention is preferably a multivalent antigen binding protein, more preferably one which comprises a single polypeptide chain having the first and second domain binding units connected in series. Optimally, the binding reagent comprises a bivalent antigen binding protein.

The domain binding units which comprise the multivalent binding protein are preferably heavy chain variable domains derived from any immunoglobulin naturally devoid of light chains, such that the antigen-binding site is located exclusively in the heavy chain variable domain. Preferably, the heavy chain variable domains are derived from immunoglobulins naturally devoid of light chains such as may be obtained from camelids as described in Casterman et al., EP-A-0584421.

Heavy chain variable domains derived from an immunoglobulin naturally devoid of light chains 'having a 'determined antigen specificity may conveniently be obtained by screening expression libraries of cloned fragments of genes encoding camelid immunoglobulins generated using conventional techniques, as described, for example, in Casterman et al., EP-A-0584421 and Frenken et al., WO 99/23221.

The multivalent antigen-binding proteins may be formed by linking together the domain binding units in series, such that each single domain binding unit is linked to at least one other variable domain. The individual domain binding unit may be linked sequentially by means of peptide linkers, conveniently flexible peptide linkers which allow the domains to flex in relation to each other such that simultaneous binding to multiple antigenic determinants may be achieved. It will be appreciated that the binding of the linker to the individual domain binding unit will be such that it does not affect the binding capacity of the domain antigen binding site. Any peptide linker which permits the domain binding units components to be linked in such a way that each variable domain retains the binding specificity of the whole immunoglobulin from which it is derived may suitably be used. Such linkers include, e.g., peptides derived from known proteins, such as glucoamylase, cellobiohydrolase, or cell wall proteins (CWP), or synthetic peptides. The linker may suitably comprise from 1 to 400 or more amino acid residues; more preferably, the peptide linker comprises from 5 to 20 amino acid residues.

In another preferred embodiment of the invention, the individual domain binding units may be connected directly in series without any intervening linker. In this way, the binding sites in the multivalent binding proteins utilised in the invention are held in much closer proximity to each other than would be the case in the whole immunoglobulin from which the immunoglobulin fragments are derived.

The binding reagent utilised in the present invention is preferably a labelled binding reagent. Alternatively, the label may reside on the analyte receptor or the reference ligand. The label allows for the first complex (i.e. the bound binding reagent, analyte and analyte receptor) to provide a test signal which is directly related to the amount of analyte in the sample. The label further allows for the second complex .(i.e. the binding reagent and the reference ligand) to provide a reference signal which is directly related to the amount of binding reagent available in the assay for binding to the analyte, and which should be substantially independent of the amount of analyte in the sample. By taking the ratio of the test signal to the reference signal, the level of analyte in the sample can be readily determined, such level being independent of the level of binding reagent contacted with the sample. Thus, mitigation of the impact on assay results of environmental and non-specific factors such as temperature, sample viscosity, ionic strength of the sample and the like, can be achieved.

The test and reference signals can be detected from the test and reference surfaces by any known conventional means. This includes evaluation by the naked eye, or if more precise measurements are desired, by appropriate instrumentation. Instrumentation is particularly suitable when the reference or test signal is measured by the amount of mass of complex at the reference or test surface.

In the preferred embodiment of the invention, the label that is conjugated to the binding reagent can be any entity the presence of which can be readily detected. Preferably the label is a direct label, such as the those described in detail in May et al., U.S. Patent 5,656,503. Direct labels are entities which, in their natural state, are readily visible either to the naked eye, or with the aid of an optical filter and/or applied stimulation, e.g. UV light to promote fluorescence. Examples include radioactive, chemiluminescent, electroactive (such as redox labels), and fluorescent compounds. Direct particulate labels, such as dye sols, metallic sols (e.g. gold) and coloured latex particles, are also very suitable and are, along with fluorescent compounds, preferred. Of these options, coloured latex particles and fluorescent compounds are most preferred. Concentration of the label into a small zone or volume should give rise to a readily detectable signal, e.g. a strongly coloured area.

Indirect labels, such as enzymes, e.g. alkaline phosphatase and horseradish peroxidase, can also be used, but these usually require the addition of one or more developing reagents such as substrates before a visible signal can be detected. Hence, they are less preferred. Such additional reagents can be incorporated in a solid support of an assay device such that they dissolve or disperse when a liquid sample is applied. Alternatively, the developing reagents can be added to the sample before application of the sample to the solid support.

Conjugation of the label to the binding reagent can be by covalent or non-covalent (including hydrophobic) bonding, if desired, or by adsorption. Techniques for such conjugation are commonplace in the art and may be readily adapted for the particular binding reagent and label utilised in the present invention. In the preferred embodiment wherein the label is a coloured latex particle, the label is preferably conjugated to the binding reagent through adsorption. Where the label is a fluorescent compound, it is preferred that the label be conjugated to or constructed as part of the binding reagent.

The analyte receptor utilised in the assays of the present invention may be any compound capable of binding to the analyte of interest, more preferably a compound which exhibits a binding specificity for the analyte of interest. The analyte receptor should bind to a different epitope on the analyte than that bound by the binding reagent, and should be such that minimal steric or other interference results between the two binding events.

In a preferred embodiment, the analyte receptor is an antibody, preferably a monoclonal antibody, though it is specifically contemplated that polyclonal antibodies may also be used. Antibodies may additionally include any functional antibody'fragments such as Fab, Fv, and even smaller units such as heavy chain variable fragments (also known in the art as HCV or VHH fragments), which may be prepared by biochemical or antibody engineering methods from known whole antibodies or indirectly from libraries of antibodies or antibody-like molecules (see, for example, Verhoeyen and Windust, *Advances in Antibody Engineering in Molecular Immunology: Frontiers in Molecular Biology,* 2nd Ed., published by Oxford University Press, pp. 283-325 (Oxford, 1995)).

Polyclonal antibodies and monoclonal antibodies may be prepared by any suitable known procedure, including those described in Price et al, Principles and Practice of Immunoassays, 2nd Ed., published by Macmillan Publishers Ltd (London, 1997).

Other entities capable of serving as an analyte receptor include single or multi-chain polypeptides which are capable of binding to the analyte. These may be prepared by any conventional methods known in the art.

The reference ligand utilised in the assay of the invention may be any compound capable of being bound by the binding reagent. This will include any antigenic compounds for which the binding reagent has binding specificity (including the particular analyte for which the assay is designed), as well as any antibody or functional fragment thereof, having a binding specificity to the binding reagent. Preferably, the reference ligand has no specific binding affinity to the analyte or binding reagent. Thus, it may include antigenic compounds, or antibodies (or fragments thereof), which contain a particular epitope to which the binding reagent can bind. Optimally, the reference ligand is a non-antibody antigen distinct from the analyte of interest.

Contact of the sample with the binding reagent and analyte receptor, and with the reference ligand, to form the first and second complexes,' respectively, may be accomplished by any conventional method. Exemplary methods range from capillary action, which occurs in conventional strip containing test devices, to simple diffusion of one or more of the components in a solution to the location of the other components, which occurs in certain forms of ELISA-type immunoassays and Energy Transfer Immunoassays ("ETI"). In such latter forms of immunoassays (i.e. ETIs), the test and reference zones can be such that they are not associated with any form of support. More typically, though, the test zones and reference zones (for all embodiments of the invention) are located on one or more supports so that, in essence, the test zone is test surface to which the binding reagent is irreversibly immobilised, and the reference zone is a reference surface to which the reference ligand is irreversibly immobilised.

If a first and second support is used for the reference zone and test zone, respectively, they may be substantially similar or identical in nature, or they may be different. The test zone will have located thereon an irreversibly immobilised analyte receptor, and the reference zone will have located thereon an irreversibly immobilised reference ligand. By "irreversibly immobilised" it is meant incorporated on or bound to the support in such a way as to prevent its migration when it (or the support) is moist. Irreversible immobilisation of the analyte receptor or reference ligand may be accomplished in any. one of a number of ways, each of which will be apparent to the person skilled in the art.

Where the reference ligand and analyte receptor are bound to supports, the invention benefits from the additional advantage of being capable of providing stronger reference signals relative to many of the internally referenced assays heretofore described in the art. This advantage flows from the fact that the assays of the present invention rely on the binding activity of the binding reagent in solution to generate the reference signal, rather than on the binding activity of an antibody-type molecule bound to the reference surface. Thus, in circumstances where a significant amount of reference ligand denatures as a result of being coated to a support, the reference signal in the present invention will be minimally affected, especially if the binding reagents specificity is designed for an epitope of the reference ligand which is not affected by the denaturing. In known internally referenced assays, by contrast, any denaturing of the antibody-type molecule on the reference surface will likely result in a concomitant decrease in binding at the surface, and hence a lower reference signal.

Solid supports which may be utilised in the invention are well' know and include, for example, synthetic plastics materials, microtitre assay plates, latex beads, filters comprising cellulosic (e.g. nitrocellulose) or synthetic polymeric materials, glass or plastic slides, dipsticks, capillary fill devices and the like.

The analytical test device of the invention incorporates a first and second support as exemplified above. The particulars of the device can be varied depending on the precise nature of the assay being performed. For example, in one embodiment, the first and second support may be physically very close to each other (in the order of a millimeter or so apart). In another embodiment, the device may comprise a capillary-fill test device in which a liquid sample may be drawn into a device by capillary action along a suitably-proportioned capillary inlet. Capillary-fill devices which may be adapted for use in the present invention are disclosed, for example, in Shanks et al., U.S. Patent 5,141,868, Shanks et al., EP-A-0422708, and Birch et al., EP-B-0274215.

In yet another embodiment, the first support comprises a synthetic plastic peg which is coated with either the binding reagent or the analyte receptor, and which is proportioned to fit within a well of a microtitre plate, there being only a small separation between the peg and the sides of the well. The second support is formed by the microtitre plate, the well also being coated with either the binding reagent or the analyte receptor, but wherein the binding reagent and analyte receptor are not both coated onto the same support. In this particular embodiment, the contact of the sample with the binding reagent can occur at substantially the same time that the sample is contacted with the reference ligand, which is preferred as it provides a distinct advantage over certain other forms of devices in that any timing-related assay variability can be minimised. Ideally, the sample is contacted with the binding reagent and the reference ligand at the same time.

Other devices, such as those described in May et al., U.S. Patent 5,622,871 and May et al., U. S. Patent 5,656,503 are also suitable for practice of. the immunoassays of the invention. If used, these devices preferably comprise a hollow elongated casing containing the solid support, which most typically is a dry porous carrier. The solid support communicates indirectly with the exterior of the casing via a bibulous fluid sample receiving member which may or may not protrude from the casing, the solid support and the sample receiving member being linked so as to allow for the fluid sample to migrate between the two by capillary action, the solid support having a zone wherein the binding reagent is reversibly immobilised -- that is, the binding reagent is immobilised on the dry solid support but becomes freely mobile on or within the solid support when the support becomes moist. Such reversible immobilisation of the binding reagent in the zone may be accomplished in any one of a number of known ways (e.g. as described in, for example, Taylor et al. *Protein Immobilisation,* published by Marcel Dekker, Inc., 1991). Specifically, it is preferred that such immobilisation occur by adsorption to the support, as described in May et al., U.S. Patent 5,622,871.

Spatially distant along the solid support from the sample receiving member are the test and reference zones. As previously described, immobilisation of the analyte receptor and reference ligand may be accomplished by any number of known means. The analyte receptor or reference ligand may be chemically coupled to the support using, for example, CNBr, carbonyldiimidazole, or tresyl chloride. Alternatively, various "printing" techniques may be used. These include application of liquid binding reagents by micro-syringes, direct printing, ink-jet printing, and the like. Chemical or physical treatment of the support prior to application of the binding reagent is also specifically contemplated, as such may facilitate immobilisation.

The casing in such devices is typically constructed of opaque or translucent material incorporating at least one aperture through which the analytical result may be observed, preferably visibly observed by the naked eye.

Such devices can be provided to clinical laboratories or as kits suitable for home use, such kits comprising one or more devices individually wrapped in moisture impervious wrapping and packaged together with appropriate instructions to the user.

The sample receiving member can be made from any bibulous, porous or fibrous material capable of absorbing liquid rapidly. The porosity of the material can be unidirectional (i.e. with pores or fibres running wholly or predominantly parallel to an axis of the member) or multidirectional (omnidirectional, so that the member has an amorphous sponge-like structure). Porous plastics material, such as polypropylene, polyethylene (preferably of very high molecular weight), polyvinylidene fluoride, ethylene vinylacetate, acrylonitrile and polytetrafluoro-ethylene can be used. It can be advantageous to pre-treat the member with a surface-active agent during manufacture, as this can reduce any inherent hydrophobicity in the member and therefore enhance its ability to take up and deliver a moist sample rapidly and efficiently. Porous sample receiving members can also be made from paper or other cellulosic materials, such as nitrocellulose. Preferably the material comprising the sample receiving member should be chosen such that the porous member can be saturated with liquid sample within a matter of seconds. The liquid must be capable of permeating freely from the porous sample receiving member into the solid support.

The solid support in such devices is preferably a dry porous carrier. It may be made of separate strips or sheets and, like the sample receiving member, can be constructed from any material capable of allowing the liquid sample to migrate through a portion of its length by, preferably, capillary action. The support should allow for the immobilisation of the analyte receptor and reference ligand on its surface, and should not interfere with the binding reactions which form the first and second complexes.

The solid support may have associated with it an absorbent "sink" which will facilitate capillary action of fluid up the length of the support, and will provide a means by which to avoid flooding of the test device by application of excess sample. Specific materials for and applications of sinks are conventional in the art and may be readily applied to the devices of the present invention.

The invention can be better appreciated by reference to the following specific examples. They are intended to be illustrative and not exhaustive of the methods and devices of the invention.

### Examples

These examples demonstrate how binding reagents having two distinct binding specificities can be utilised in immunoassays to correct for deliberately introduced assay variation. It will be appreciated that it is possible for those skilled in the art to design alternative construction routes for the binding reagents (and other components) utilised in these examples.

### Example 1 Correcting Assay Variation Using a Llama Bi-Head Green Fluorescent Protein Binding Reagent

This example shows that a llama bi-head green fluorescent protein binding reagent which contains two binding sites, one for human chorionic gonadotrophin (hCG, the analyte) and one for reactive red 6 dye (RR6, the reference ligand) can correct for deliberately introduced assay variation by taking a ratio of the resulting assay binding signals. In this example, the binding of the binding reagent is detected by virtue of its green fluorescent protein domain using flow cytometry.

### 1.1 Construction, expression and purification of the binding reagent

The construction, expression and purification of the llama bi-head green fluorescent protein binding reagent (GFP-HCV llama bi-head green fluorescent fusion protein) utilised in this example was done in the following manner.

### 1.1.1 Construction of the binding reagent expression vector

The construction of the final pPIC-HIS6-GFP-HCV21-myc expression vector involved several cloning steps and the following plasmids as starting material:

| | |
|---|---|
| pEGFP-N2 | (CLONTECH, Genbank accession nr: U57608) |
| pPIC9 | (Invitrogen, EMBL Accession: Z46233) |
| pUC19 | (New England Biolabs, GenBank Accession: (published May 14, 1999 X02514) |
| pPIC.HCV21. | (Obtained in a manner as described in detail in Frenken et al., WO 99/23221, published May 14, 1999 (EMBL Accession CAA15419 and EMBL Accession CAA15409). |

To allow the expression and secretion of the HIS6-GFP-HCV21-myc fusion constructs in *P.pastoris,* the gene encoding the HIS6-GFP-HCV21-myc construct was fused to the alpha-mating factor leader sequence in the. commercially available *P.pastoris* expression vector pPIC9 (Invitrogen). The construction of the final expression vectors involved several cloning steps resulting in two intermediate vectors, pPIC9-HIS6 and pPIC-HIS6-GFP. For pPIC9-HIS6 the XhoI/SnaBI fragment of the leader peptide in pPIC9 was removed and replaced with a synthetic XhoI/SnaBI fragment, replacing the deleted leader sequence fragment, plus fusing it to a 6xHIS sequence.
Synthetic insert of pPIC9-HIS6:

The synthetic linker was constructed by annealing the synthetic oligonucleotides PCR.529 and PCR.530

The expression vector pPIC9-HIS6-GFP was constructed by opening the pPIC9-HIS6 vector with SnaBI/NotI, thus removing the polylinker sequence from the vector, and replacing it with the SmaI/NotI GFP encoding gene sequence from pEGFP-N2.

The final expression vector pPIC9-HIS6-GFP-HCV21-myc was constructed in a 3 point ligation reaction linking the XhoI/XbaI HIS6-GFP PCR fragment from pPIC9-HIS6-GFP (using PCR.393 and PCR.689) to the NheI/NotI HCV21-myc fragment from pPIC9-HCV21-myc, and cloning it into XhoI/NotI opened pPIC9.

### 1.1.2 Expression and purification of the binding reagent

BglII opened pPIC-HIS6-GFP-HCV21-myc was used to transform *P.pastoris* cells as described as follows:

*P.pastoris* GS115 cells were grown overnight at 30°C in 500ml YDP medium (1% Yeast Extract, 2% Peptone, 1% Glucose) to OD₆₀₀=1.4. The cells were spun and the pellet was washed with steril distilled water before resuspending in 100ml KDTT buffer (50 mM Potassium Phosphate pH7.5, 25 mL DTT). After 15 min. inbucation at 37°C the cells were pelleted (3 min. 3000 rpm) and resuspended in 100ml ice-cold STM buffer (92.4g Glucose/1, 10 mM Tris.HCL pH7.5, 1 mL MgCl₂). After 5 washes with this buffer the cell pellet was resuspended in a final volume of 0.5ml STM buffer. Approximately 2-5µl H₂O (digested pPIC9 constructs: DNA purified via Phenol/Chloroform extractions and EtOH precipitation) was mixed with 70µl of fresh competent *P.pastoris* cells (on ice). The cells were electroporated in 0 0.2cm cuvette at 1.5kV, 400, 25µF in a BioRad Gene-Pulser. Immediately after electroporation, 1ml of YPD medium was added to the cells. After recovery for 1 h at 30°C, the cells were pelleted and resuspended in 200µl 1M Sorbitol and plated out onto MD plate (1.34% YNB, 4x10⁻⁵% Biotin, 1% Glucose, 0.15% Agar). Colonies formed by transformed cells (His⁺) were visible within 48 hours incubation at 30°C. Transformed *P.pastoris* cells GS115 were selected essentially as recommended by the Invitrogen Pichia Pastoris Expression Manual: The plates containing the His⁺ transformants were used to screen for the Mut⁺ and Mut^{S} phenotype as follows: Using sterile toothpicks, colonies were patched on both an MM plate (1.34% YNB, 4x10⁻⁵% Biotin, 0.5% MeOH, 0.15% Agar) and an MD plate, in a regular pattern, making sure to patch the MM plate first. Approximately 100 transformants were picked for each construct. After incubating the plates at 30°C for 2-3 days the plates were scored. Colonies that grow normally on the MD plates but show little or no growth on the MM plates wree classified as Mut^{S} clones.

Transformed and selected *P.pastoris* clones were induced to 'express the binding reagent using the protocol outlined below:
1. Using a single colony from the MD plate, inoculate 10ml of BMGY (1% Yeast Extract, 2% Peptone, 100mM potassium phosphate pH6.0, 1.34% YNB, 4x10⁻⁵% Biotin, 1% Glycerol) in a 50ml tube.
2. Grow at 30°C in a shaking incubator (250 rpm) until the culture reaches an OD₆₀₀=2-8.
3. Spin the cultures at 2000g for 5 min and resuspend the cells in 2ml of BMMY medium (1% Yeast Extract, 2% Peptone, 100mM potassium phosphate pH6.0, 1.34% YNB, 4x10⁻⁵% Biotin, 0.5% Glycerol).
4. Return the cultures to the incubator.
5. Add 20µL of MeOH to the cultures after 24 hours to maintain induction. After 48 hours harvest the supernatant by removing the cells by centrifugation.

The crude supernatants were tested for the presence of HCV bi-head fragment via analysis on 12% acrylamide gels using the Bio-Rad mini-Protean II™ system.

Bispecific binding activity shown via ELISA as follows:
1. 96 well ELISA plates (Greiner HC plates) were activated overnight at 37°C with 200 µl/well of the BSA-RR6 conjugate (see example 1) in PBS.
2. Following one wash with PBST the wells were incubated for 1 hour at 37°C with 200µL blocking buffer per well. Blocking buffer:1% BSA in PBS-T
3. Serial dilutions of test samples (100µL) were mixed with equal volumes of blocking buffer and added to the sensitised ELISA wells. Incubated at 37 C for 1-2 hours.
4. 200 pL hCG-AP conjugate in blocking buffer was added to each well in which the hCG was coupled to the alkaline phospathase via glutaraldehyde coupling.
5. Following one wash with PBST captured hCG-AP was detected by adding 100µl/well pNPP substrate (1mg/mL pNPP in 1M diethanolamine/1mM MgCl

The crude supernantants were tested for the presence of GFP (green fluorescent protein) activity by diluting the supernatants (10 ml) to 100 ml in PBSTA. These were then analysed on a Perkin Elmer fluorimeter with excitation at 488 nm and emission detected at 509 nm.

The culture supernatant (200 mL, pH 6-8) was clarified through a 0.45m low protein binding cellulose acetate filter (Nalge Nunc Intl.), applied to a Ni-NTA Superflow column (5mL, Qiagen Ltd, UK) at 2 mL/min, and washed with PBSA until the absorbance at 280 nm reached baseline. Elution with a linear gradient of 0 - 500mM imidazole over 5 column volumes was followed by immediate buffer exchange by passage down a column of G-25 Sepadex (150mL bed volume, Pharmacia) pre-equilibrated with PBSA, collecting 4mL fractions. Peak fractions were assayed by SDS-PAGE and ELISA then combined and freeze dried in aliquots.

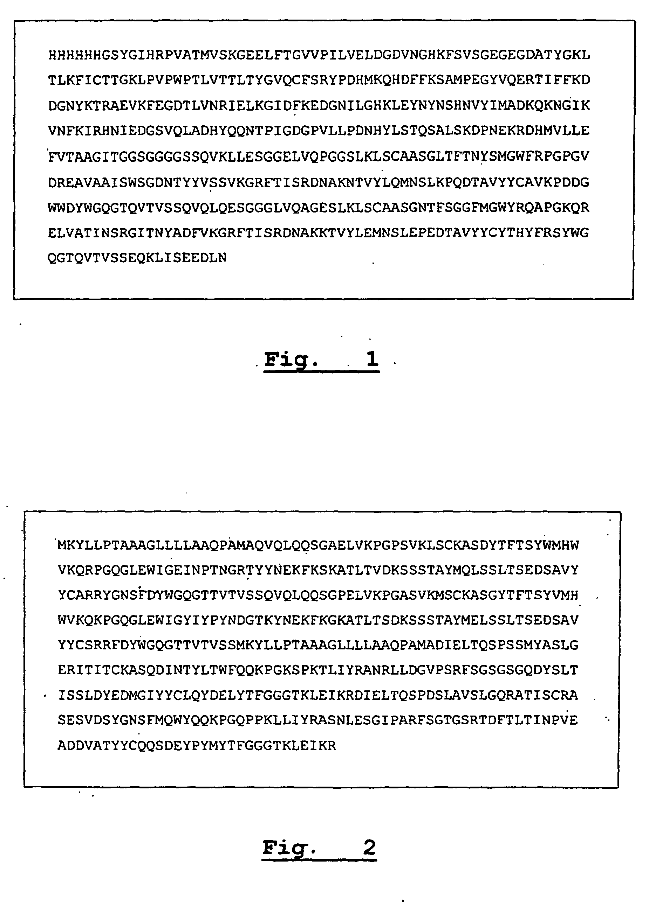

Specifically, the binding reagent constructed by the foregoing process comprised the amino acid sequence as described in Figure 1 (SEQ ID NO:1)

### 1.2 Preparation of a reference ligand

A reactive red 6-bovine serum albumin conjugate reference ligand (RR6-BSA) was prepared by incubating 200 µl reactive red 6 (10 mg/ml) with 1 ml bovine serum albumen (10 mg/ml) in an end over end mixer for 3 hours at room temperature (20°C). Following this, 200 µl ethanolamine (1 M) was added and the solution was incubated for a further 15 minutes. Free unbound reactive red 6 was removed from the bovine serum albumen fraction by passing 0.75 ml of the solution down a Pharmacia PD10™ desalting column pre-equilibrated with phosphate buffered saline containing 0.01 % sodium azide and eluted with equilibration buffer. Fractions (1ml) were collected and utilised in this and subsequent examples.

### 1.3 Preparation of an analyte receptor

A monoclonal antibody having binding specificity to hCG and representing the analyte receptor (MAb 1140) was prepared according to procedures known in the art. A representative method for generating monoclonal antibodies is described by Gani *et al* J. Steroid Biochem. Molec. Biol. 1994, vol. 48, pp. 277-282) and this method can be adapted to produce a relevant antibody having a binding specificity to hCG. A suitable monoclonal antibody can be selected on the basis of its relative affinity and specificity for the analyte and analyte analogues. This can be undertaken, for example, by performing standard kinetic determinations using a Biacore™ 2000 biosensor (Biacore AB, Sweden), as described in the manufacturer's protocol (Applications handbook, Biacore AB), using a panel of closely related analogues. MAb is also available commercially and can be obtained from Calbiochem - Novabiochem (UK) Ltd, Nottingham, UK.

### 1.4 Formation of test and reference zones

Formation of test and reference zones by adsorption of the analyte receptor and reference ligand to latex surfaces was done in the following manner. A 3µm latex adsorbed with RR6-BSA was prepared as follows. Latex stock solution (500 µl of 1 % solids) was pipetted into a round bottomed eppendorf and to this 500 µl 10 mM borate buffer, pH 8.5 containing 0.01 % merthiolate was added. The solution was mixed and centrifuged on a bench top eppendorf centrifuge for 10 min at room temperature. The supernatant was removed and the pellet briefly vortexed. In a separate eppendorf, 100 µl RR6-BSA conjugate solution was added to 900 µl borate buffer and the resulting solution was mixed and added to the latex pellet. The latex - RR6-BSA solution was vortexed, sonicated with a sonic probe for 10 seconds and then incubated for 1 hour at room temperature (20°C) on an end-over end mixer After 1 hour 50 µl of a 200 mg/ml bovine serum albumen solution was added and the latex solution incubated for a further 30 min. The latex beads were centrifuged for 10 min at room temperature in an eppendorf centrifuge and the pellet resuspended in 1 ml of borate buffer. A reference zone representing the latex beads adsorbed with RR6-BSA in the amount of 10 µl (0.5% solids) was separated from this suspension and stored at 4 °C until required.

A 3µm latex, obtained as described above, was adsorbed with a MAb 1140 was prepared as follows. Latex stock solution (500 µl of 1 % solids) was pipetted into a round bottomed eppendorf and centrifuged on a bench top eppendorf centrifuge. The supernatant was removed and. to this 500 µl 10 mM borate buffer, pH 8.5 containing 0.01 % merthiolate was added. The solution was mixed and centrifuged as described above. The supernatant was removed and the pellet briefly vortexed. In a separate eppendorf 1 ml of a 1 mg/ml MAb 1140 solution made up in borate buffer was prepared and the resulting solution was mixed and added to the latex pellet. The latex - MAb 1140 solution was vortexed and sonicated with a sonic probe for 10 seconds. To this solution, 200 µl of 95 % (v/v) ethanol 0.5 % (w/v) sodium acetate was added and the resulting solution vortexed. After 2 hours of mixing at 37 °C in an end-over-end rotary mixer, 50 µl of a 200 mg/ml BSA solution was added and the latex solution incubated for a further 30 min. The latex beads were centrifuged for 10 min at room temperature in an eppendorf centrifuge and the pellet resuspended in 1 ml of borate buffer. A test zone representing the latex beads adsorbed with MAb 1140 in the amount of 10 µl (0.5% solids) was separated from this suspension and stored at 4 °C until required.

### 1.5 Internally referenced assay for the detection of hCG

A test zone as prepared and described above was exposed to a solution containing an analyte, hCG (10 µl of 20 IU/ml), and a 20 µl solution containing a binding reagent (i.e. llama bi-head green fluorescent protein as prepared and described above) in a concentration as set forth in Table 1, with the volume being made up to 50 µl with phosphate buffered saline. The test zone was incubated in the solution for 1 hour at room temperature (20°C).

A reference zone as prepared and described above was subjected to the same conditions as the test zone. Following the 1 hour incubation period, both the test and reference zones were quantified for fluorescence 'using a Coulter Elite™ flow cytometer with an argon laser (mean fluorescent units were measured on the latex bead populations).

The results are described in Table 1. As is clearly evident, in each test performed the assay variation as measured by the coefficient of variation was reduced by utilisation of internal referencing; and, in particular, by utilisation of internal referencing that made use of a reference surface and a binding reagent having binding specificity for the both the analyte and the reference ligand.

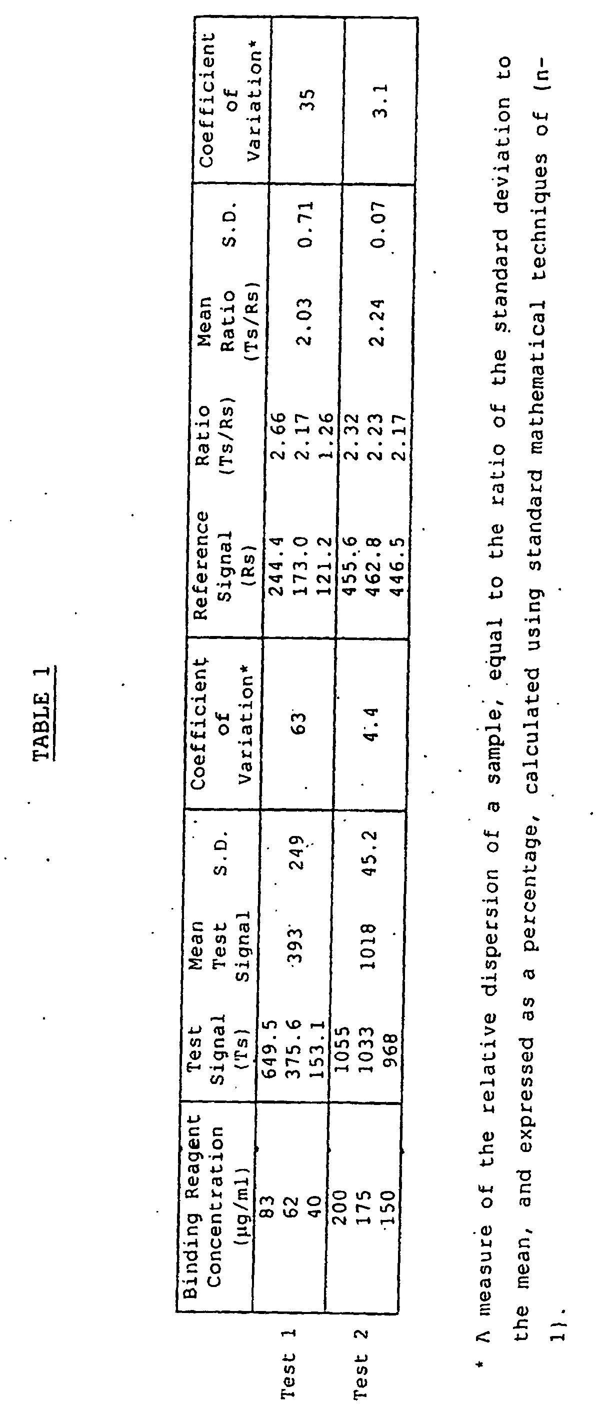

### Example 2 Correcting Assay Variation Using a Double-Head Antibody Binding Reagent

This example shows that a double-head antibody binding reagent which contains two binding sites, one for human chorionic gonadotrophin (hCG, the analyte) and one for glucose oxidase (GOx, the reference ligand) can correct for deliberately introduced assay variation by taking a ratio of the resulting binding signals. In this example, the binding of the binding reagent is detected by virtue of its inherent mass using a surface plasmon resonance biosensor.

### 2.1 Construction, expression and purification of the binding reagent

The construction, expression and purification of the double-head binding reagent utilised in this example was done in a manner as described in Davis et al., WO 97/14719, particularly with respect to the construction of the GOSA.T construct, except that the anti-S.sanguis specific VH4715 and VL4715 domains were replaced by the anti-hCG specific VH3299 and VL3299 domains (described in WO96/27612 as FvKC-II, linked via a BstE11-Sac1 linker fragment) by conventional means (as exemplified in Sambrook et al. (1989) Molecular Cloning, A Laboratory Manual (2^{nd} Edition, Cold Spring Harbour Laboratory Press)), thus yielding an anti-hCG/anti-GOx double-head binding reagent comprising the amino acid sequence identified in Figure 2 (SEQ ID NO:2).

### 2.2 Preparation of a test and reference zone on a biosensor chip

A CM5 biosensor chip (Biacore AB) was coupled with reference ligand, GOx, and analyte receptor MAb 1140 (as prepared in Example 1.3). in the following manner. The chip was docked in a Biacore™ 2000 biosensor (Biacore AB) and a sensorgram was run as follows. The flow path was set to flow across flow cells 1, 2, 3 and 4 and the flow rate of hepes buffered saline (HBS, Biacore AB) was set to 10 µl/min. The sensor chip surface was then activated by two sequential injections of 60 µl of a 1, ethyl-3-[dimethylaminopropyl]carbodiimide (EDC) N-hydroxysuccinimide (NHS) activating solution made up as described by the manufacturer (Biacore AB). The flow path was then changed to flow across flow cell 2 and two sequential injections of GOx (*Aspergillus niger*, obtained from Novo Nordisk, Copenhagen, Denmark) diluted to 100 µg/ml in 10 mM sodium citrate buffer pH 4 were performed. The flow path was then changed to flow across flow cell 3 and injections (3 x 20 µl and 1 x 40 µl) of a MAb 1140. The flow path was once again changed to flow across flow cells 1, 2, 3 and 4 and excess activated binding sites were blocked by two 60 µl injections of 1 M ethanolamine.

The amount of GOx coupled to flow cell 2 of the biosensor chip was 2770 Response Units ("RU", as measured on a Biacore™ 2000 biosensor). The amount of MAb 1140 coupled to flow cell 3 of the biosensor chip was 2570 RU. No other flow cells had coupled thereto any GOx or MAb 1140. Flow cells 1, and 4 represented control surfaces. Flow cell 2 represented the reference zone whereas flow cell 3 represented the test zone.

### 2.3 Internally references assay for the detection of hCG

The double-head antibody binding reagent prepared in paragraph 2.1 was diluted with HBS in the following ratios (19:1, 18:2, 17:3; bi-head : HBS). Therefore, these solutions varied slightly from the non-diluted solution and from each other in the amount of binding reagent they contained.

A Biacore method was set up to perform the following. The flow rate of HBS was set to 10 µl/min and the flow path set to cross flow cells 1, 2, 3 and 4. Twenty microlitres hCG (10 IU/ml in HBS was injected across the sensor surfaces. Only the surface at flow cell 3 captured any hCG (this was the surface with coupled analyte receptor MAb 1140). Twenty microlitres binding reagent (non-diluted solution) was then injected across all flow cells of the sensor chip. Capture of the binding reagent occurred at the surface of flow cell 2 (coupled with reference ligand, GOx) and also at the surface of flow cell 3 (now containing captured analyte, hCG). The amount of binding reagent binding was determined in RU. The surfaces were then regenerated by an injection of 5 µl 10 mM HCl. Injections of hCG (10 IU/ml) and double-head binding reagent were repeated three times but this time using the 19:1, 18:2 and 17:3 ratios of the binding reagent. This gave four values for binding reagent capture. Flow cells 1 and 4 exhibited no capture. Flow cells 2 and 3 exhibited capture of the binding reagent. The foregoing test (Test 1) was repeated using 5 IU/ml hCG to yield Test 2.

The results, in terms of coefficient of variation, are shown in Table 2. As with Example 1, in each test performed the assay variation as measured by the coefficient of variation was reduced by utilisation of internal referencing; and, in particular, by utilisation of internal referencing that made use of a reference surface and a binding reagent having binding specificity for the both the analyte and the reference ligand.

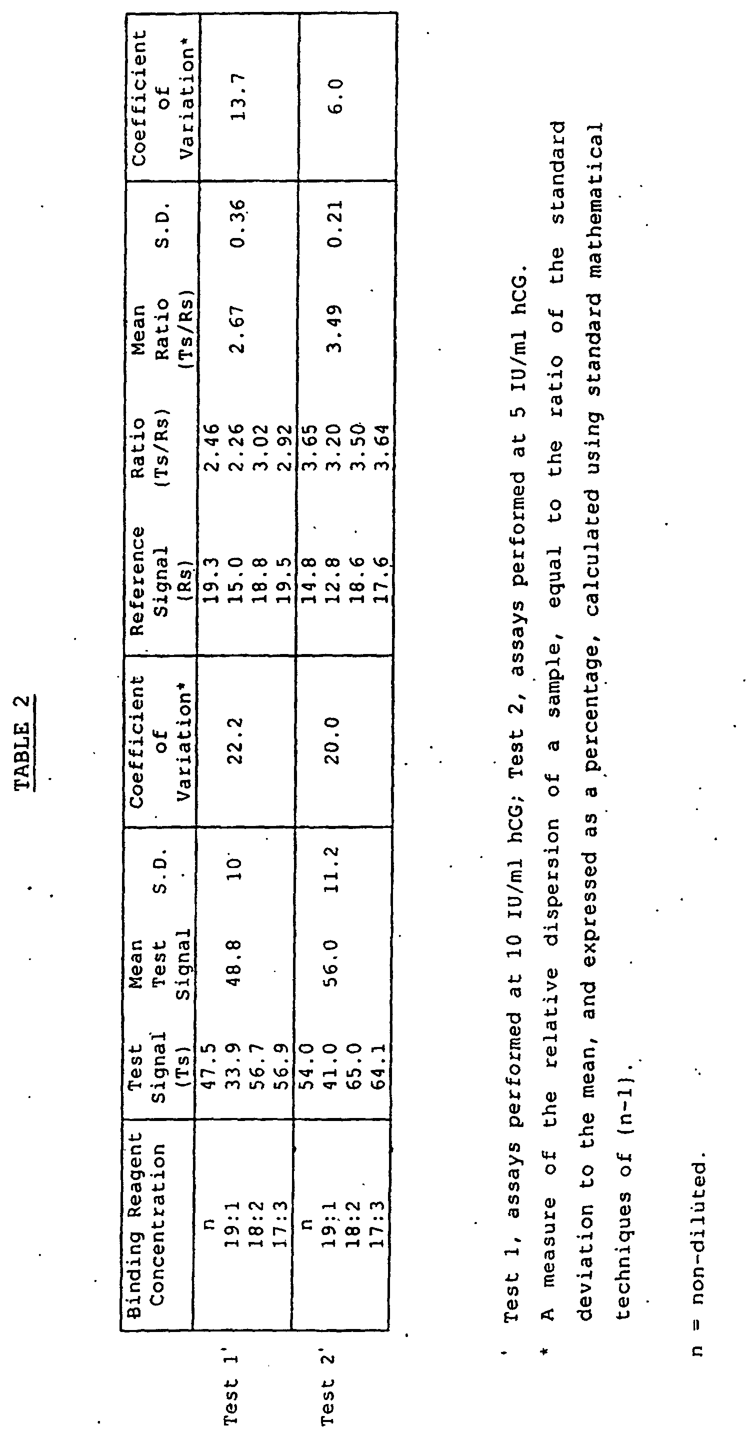

## Claims

1. A method of detecting the presence of an analyte in a sample, the method comprising:
a) contacting the sample with a binding reagent and an analyte receptor to form a first complex comprising the binding reagent, analyte, and analyte receptor, the first complex providing a test signal, wherein the analyte receptor is located within a test zone and is capable of binding to the analyte, and the binding reagent has a binding specificity for the analyte;
b) contacting the sample with a reference ligand to form a second complex comprising the binding reagent and reference ligand, the second complex providing a reference signal, wherein the reference ligand is located within a reference zone, and further wherein the binding reagent has a binding specificity for the reference ligand; and
c) detecting from the test and reference zones the test and reference signals, whereby the ratio of the test signal to the reference signal, when taken, defines the level of analyte in the sample, such level being independent of the level of binding'reagent contacted with the sample.

2. A method according to claim 1 wherein the test zone is a test surface to which the analyte receptor is irreversibly immobilised, and wherein the reference zone is a reference surface to which the reference ligand is irreversibly immobilised.

3. A method according to claim 1 or claim 2 wherein the binding reagent is selected from an antibody or a multivalent antigen binding protein, the antibody and multivalent antigen binding protein each comprising a first and second domain binding unit, wherein the first domain binding unit provides a binding specificity for the analyte and the second domain binding unit provides a binding specificity for the reference ligand.

4. A method according to any one of claims 1 to 3 wherein the binding reagent is a multivalent antigen binding protein.

5. A method according to any one of claims 1 to 4 wherein the binding reagent is a multivalent antigen binding protein comprising a single polypeptide chain having the first and second domain binding units connected in series.

6. A method according to any one of claims 1 to 5 wherein the binding reagent comprises a bivalent antigen binding protein.

7. A method according to any one of claims 3 to 6 wherein the single domain binding units are heavy chain variable domains derived from an immunoglobulin naturally devoid of light chains.

8. A method according to any one of claims 3 to 7 wherein the single domain binding units are heavy chain variable domains derived from a Camelid immunoglobulin.

9. A method according to any one of claims 1 to 8 wherein the binding reagent is a labeled binding reagent.

10. A method according to claim 9 wherein the label is a coloured latex particle or fluorescent compound.

11. A method according to any one of claims 1 to 10 wherein the sample is selected from the group consisting of urine, serum, saliva, cervical or urethral fluid.

12. A method according to any one of claims 1 to 11 wherein contacting the sample with the binding reagent occurs at substantially the same time as the sample is contacted with the reference ligand.

13. A method according to any one of claims 1 to 12 wherein the analyte receptor is an antibody having binding specificity to the analyte.

14. A method according to any one of claims 1 to 13 wherein the reference ligand is an antigen for which the binding reagent exhibits a binding specificity.

15. An analytical test device for detecting the presence of an analyte in a liquid biological sample, the test device comprising a binding reagent, an analyte receptor, a reference ligand and first and second solid supports, the second solid support comprising a test zone, the test zone having irreversibly immobilised thereon the analyte receptor which, when bound to the analyte and binding reagent, forms a first complex which provides a test signal, the first solid support comprising a reference zone, the reference zone having irreversibly immobilised thereon the reference ligand which, when bound to the binding reagent, forms a second complex which provides a reference signal which is a means by which to correct for variations in the amount of binding reagent exposed to the sample; wherein the binding reagent is selected from an antibody or a multivalent antigen binding protein, the antibody and multivalent antigen binding protein each comprising a first and second domain binding unit, wherein the first domain binding unit provides a binding specificity for the analyte and the second domain binding unit provides a binding specificity for the reference ligand.

16. An analytical test device according to claim 15 wherein the binding reagent is a multivalent antigen binding protein.

17. An analytical test device according to claim 15 or claim 16 wherein multivalent antigen binding protein comprises a single polypeptide chain having the first and second domain binding units connected in series.

18. An analytical test device according to any one of claims 15 or claim 17 wherein the binding reagent comprises a bivalent antigen binding protein and the analyte receptor is an antibody having binding specificity to the analyte.

19. An analytical test device according to any one of claims 15 to 18 wherein the single domain binding units are heavy chain variable domains derived from an immunoglobulin naturally devoid of light chains.

20. An analytical test device according to any one of claims 15 to 19 wherein the single domain binding units are heavy chain variable domains derived from a Camelid immunoglobulin.

21. An analytical test device according to any one of claims is to 20 wherein the binding reagent is a labeled binding reagent.

22. An analytical test device according to claim 21 wherein the label is a coloured latex particle or a fluorescent compound.

## Patentansprüche

1. Verfahren zum Nachweis der Anwesenheit eines Analyten in einer Probe, wobei das Verfahren umfasst:
a) in Kontakt bringen der Probe mit einem Bindungsreagenz und einem Analytrezeptor, um einen ersten Komplex zu bilden, umfassend das Bindungsreagenz, den Analyten und den Analytrezeptor, der erste Komplex liefert ein Testsignal, wobei der Analytrezeptor in einer ersten Testzone angeordnet ist und fähig ist, an den Analyten zu binden, und das Bindungsreagenz eine Bindungsspezifität für den Analyten besitzt;
b) in Kontakt bringen der Probe mit einem Referenzliganden, um einen zweiten Komplex zu bilden, umfassend das Bindungsreagenz und den Referenzliganden, der zweite Komplex liefert ein Referenzsignal, wobei der Referenzligand in einer Referenzzone angeordnet ist, und weiterhin wobei das Bindungsreagenz eine Bindungsspezifität für den Referenzliganden besitzt; und
c) Nachweisen der Test- und Referenzsignale aus den Test- und Referenzzonen, wobei das Verhältnis des Testsignals zu dem Referenzsignal, wenn es aufgenommen wird, die Menge des Analyten in der Probe definiert, diese Menge ist unabhängig von der Menge des Bindungsreagenz, die mit der Probe in Kontakt gebracht wurde.

2. Verfahren gemäß Anspruch 1, wobei die Testzone eine Testoberfläche ist, an die der Analytrezeptor irreversibel immobilisiert ist, und wobei die Referenzzone eine Referenzoberfläche ist, an die der Referenzligand irreversibel immobilisiert ist.

3. Verfahren gemäß Anspruch 1 oder Anspruch 2, wobei das Bindungsreagenz ausgewählt ist unter einem Antikörper oder einem multivalenten Antigen-bindenden Protein, der Antikörper und das multivalente Antigen-bindende Protein weisen jeweils eine erste und eine zweite Domänen-Bindungseinheit auf, wobei die erste Domänen-Bindungseinheit eine Bindungsspezifiät für den Analyten liefert und die zweite Domänen-Bindungseinheit eine Bindungsspezifität für den Referenzliganden liefert.

4. Verfahren gemäß einem der Ansprüche 1 bis 3, wobei das Bindungsreagenz ein multivalentes Antigen-bindendes Protein ist.

5. Verfahren gemäß einem der Ansprüche 1 bis 4, wobei das Bindungsreagenz ein multivalentes Antigen-bindendes Protein ist, das eine einzelne Polypeptidkette mit den ersten und zweiten Domänen-Bindungseinheiten, die in Reihe verbunden sind, umfasst.

6. Verfahren gemäß einem der Ansprüche 1 bis 5, wobei das Bindungsreagenz ein bivalentes Antigen-bindendes Protein umfasst.

7. Verfahren gemäß einem der Ansprüche 3 bis 6, wobei die Einzeldomänen-Bindungseinheiten variable Domänen der schweren Kette sind, die von einem Immunglobulin abgeleitet sind, das natürlicherweise keine leichten Ketten aufweist.

8. Verfahren gemäß einem der Ansprüche 3 bis 7, wobei die Einzeldomänen-Bindungseinheiten variable Domänen der schweren Kette sind, die von einem Camelid-Immunglobulin abgeleitet sind.

9. Verfahren gemäß einem der Ansprüche 1 bis 8, wobei das Bindungsreagenz ein markiertes Bindungsreagenz ist.

10. Verfahren gemäß Anspruch 9, wobei die Markierung ein farbiges Latexpartikel oder eine Fluoreszenzverbindung ist.

11. Verfahren gemäß einem der Ansprüche 1 bis 10, wobei die Probe ausgewählt ist aus der Gruppe bestehend aus Harn, Serum, Speichel, Gebärmutterhals- oder Harnröhrenflüssigkeit.

12. Verfahren gemäß einem der Ansprüche 1 bis 11, wobei das in Kontakt bringen der Probe mit dem Bindungsreagenz im Wesentlichen zur selben Zeit stattfindet, zu der die Probe mit dem Referenzliganden in Kontakt gebracht wird.

13. Verfahren gemäß einem der Ansprüche 1 bis 12, wobei der Analytrezeptor ein Antikörper mit einer Bindungsspezifität für den Analyten ist.

14. Verfahren gemäß einem der Ansprüche 1 bis 13, wobei der Referenzligand ein Antigen ist, für welches das Bindungsreagenz eine Bindungsspezifität zeigt.

15. Analytische Testvorrichtungen zum Nachweis der Anwesenheit eines Analyten in einer flüssigen biologischen Probe, die Testvorrichtung umfasst ein Bindungsreagenz, einen Analytrezeptor, einen Referenzliganden und erste und zweite feste Träger, der zweite feste Träger umfasst eine Testzone, die Testzone weist irreversibel an sie immobilisiert den Analytrezeptor auf, der, wenn er an den Analyten und das Bindungsreagenz gebunden ist, einen ersten Komplex bildet, der ein Testsignal liefert, der erste feste Träger umfasst eine Referenzzone, die Referenzzone weist an sie irreversibel immobilisiert den Referenzliganden auf, der, wenn er an das Bindungsreagenz gebunden ist, einen zweiten Komplex bildet, der ein Referenzsignal liefert, das ein Mittel ist, durch das Variationen in der Menge des Bindungsreagenz, die der Probe ausgesetzt ist, korrigiert werden; wobei der Bindungsreagenz ausgewählt ist unter einem Antikörper oder einem multivalenten Antigen-bindenden Protein, der Antikörper und das multivalente Antigen-bindende Protein umfassen jeweils eine erste und zweite Domänen-Bindungseinheit, wobei die erste Domänen-Bindungseinheit eine Bindungsspezifität für den Analyten liefert und die zweite Domänen-Bindungseinheit eine Bindungsspezifität für den Referenzliganden liefert.

16. Analytische Testvorrichtung gemäß Anspruch 15, wobei das Bindungsreagenz ein multivalentes Antigen-bindendes Protein ist.

17. Analytische Testvorrichtung gemäß Anspruch 15 oder 16, wobei das multivalente Antigen-bindende Protein eine einzelne Polypeptidkette mit den ersten und zweiten Domänen-Bindungseinheiten, die in Reihe verbunden sind, umfasst.

18. Analytische Testvorrichtung gemäß einem der Ansprüche 15 bis 17, wobei das Bindungsreagenz ein bivalentes Antigen-bindendes Protein umfasst, und der Analytrezeptor ein Antikörper mit Bindungsspezifität für den Analyten ist.

19. Analytische Testvorrichtung gemäß einem der Ansprüche 15 bis 18, wobei die Einzeldomänen-Bindungseinheiten variable Domänen der schweren Kette sind, die von einem Immunglobulin abgeleitet sind, das natürlicherweise keine leichten Ketten aufweist.

20. Analytische Testvorrichtung gemäß einem der Ansprüche 15 bis 19, wobei die Einzeldomänen-Bedindungseinheiten variable Domänen der schweren Kette sind, die von einem Camelid-Immunglobulin abgeleitet sind.

21. Analytische Testvorrichtung gemäß einem der Ansprüche 15 bis 20, wobei das Bindungsreagenz ein markiertes Bindungsreagenz ist.

22. Analytische Testvorrichtung gemäß Anspruch 21, wobei die Markierung ein farbiges Latexpartikel oder eine Fluoreszenzverbindung ist.

## Revendications

1. Procédé pour détecter la présence d'un analyte dans un échantillon, le procédé consistant :
a) à mettre en contact l'échantillon avec un réactif de liaison et un récepteur d'analyte pour former un premier complexe comprenant le réactif de liaison, l'analyte, et le récepteur d'analyte, le premier complexe fournissant un signal de test, dans lequel le récepteur d'analyte est situé dans une zone de test et est capable de se lier à l'analyte, et le réactif de liaison a une spécificité de liaison pour l'analyte ;
b) à mettre en contact l'échantillon avec un ligand de référence pour former un second complexe comprenant le réactif de liaison et le ligand de référence, le second complexe fournissant un signal de référence, dans lequel le ligand de référence est situé dans une zone de référence, et de plus dans lequel le réactif de liaison a une spécificité de liaison pour le ligand de référence ; et
c) à détecter à partir des zones de test et de référence les signaux de test et de référence, ainsi le rapport du signal de test au signal de référence, quand il est pris, définit le taux d'analyte dans l'échantillon, un tel taux étant indépendant du taux de réactif de liaison mis en contact avec l'échantillon.

2. Procédé selon la revendication 1, dans lequel la zone de test est une surface de test sur laquelle le récepteur d'analyte est immobilisé de façon irréversible, et dans lequel la zone de référence est une surface de référence sur laquelle le ligand de référence est immobilisé de façon irréversible.

3. Procédé selon la revendication 1 ou la revendication 2, dans lequel le réactif de liaison est choisi parmi un anticorps ou une protéine de liaison d'antigène multivalente, l'anticorps et la protéine de liaison d'antigène multivalente comprenant chacun un premier et un second motifs de liaison de domaine, où le premier motif de liaison de domaine fournit une spécificité de liaison pour l'analyte et le second motif de liaison de domaine fournit une spécificité de liaison pour le ligand de référence.

4. Procédé selon l'une quelconque des revendications 1 à 3, dans lequel le réactif de liaison est une protéine de liaison d'antigène multivalente.

5. Procédé selon l'une quelconque des revendications 1 à 4, dans lequel le réactif de liaison est une protéine de liaison d'antigène multivalente comprenant une seule chaîne de polypeptides possédant les premier et second motifs de liaison de domaine reliés en série.

6. Procédé selon l'une quelconque des revendications 1 à 5, dans lequel le réactif de liaison comprend une protéine de liaison d'antigène bivalente.

7. Procédé selon l'une quelconque des revendications 3 à 6, dans lequel les seuls motifs de liaison de domaine sont des domaines variables de chaînes lourdes provenant d'une immunoglobuline naturellement dépourvue de chaînes légères.

8. Procédé selon l'une quelconque des revendications 3 à 7, dans lequel les seuls motifs de liaison de domaine sont des domaines variables de chaînes lourdes provenant d'une immunoglobuline Camelid.

9. Procédé selon l'une quelconque des revendications 1 à 8, dans lequel le réactif de liaison est un réactif de liaison marqué.

10. Procédé selon la revendication 9, dans lequel le marqueur est une particule de latex colorée ou un composé fluorescent.

11. Procédé selon l'une quelconque des revendications 1 à 10, dans lequel l'échantillon est choisi dans le groupe formé par l'urine, le sérum, la salive, le liquide cervical ou urétral.

12. Procédé selon l'une quelconque des revendications 1 à 11, dans lequel une mise en contact de l'échantillon avec le réactif de liaison se produit pratiquement au même moment où l'échantillon est mis en contact avec le ligand de référence.

13. Procédé selon l'une quelconque des revendications 1 à 12, dans lequel le récepteur d'analyte est un anticorps ayant une spécificité de liaison à l'analyte.

14. Procédé selon l'une quelconque des revendications 1 à 13, dans lequel le ligand de référence est un antigène quant auquel le réactif de liaison présente une spécificité de liaison.

15. Dispositif de test analytique destiné à détecter la présence d'un analyte dans un échantillon de liquide biologique, le dispositif du test comprenant un réactif de liaison, un récepteur d'analyte, un ligand de référence et des premier et second supports solides, le second support solide comprenant une zone de test, la zone de test ayant immobilisée de façon irréversible sur celle-ci le récepteur d'analyte qui, quand il est lié à l'analyte et au réactif de liaison, forme un premier complexe qui fournit un signal de test, le premier support solide comprenant une zone de référence, la zone de référence ayant immobilisé de façon irréversible sur celle-ci le ligand de référence qui, lorsqu'il est lié au réactif de liaison, forme un second complexe qui fournit un signal de référence qui est un moyen permettant de corriger des variations dans la quantité de réactif de liaison exposé à l'échantillon ; dans lequel le réactif de liaison est choisi parmi un anticorps ou une protéine de liaison d'antigène multivalente, l'anticorps et la protéine de liaison d'antigène multivalente comprenant chacun un premier et un second motifs de liaison de domaine, où le premier motif de liaison de domaine fournit une spécificité de liaison pour l'analyte et le second motif de liaison de domaine fournit une spécificité de liaison pour le ligand de référence.

16. Dispositif de test analytique selon la revendication 15, dans lequel le réactif de liaison est une protéine de liaison d'antigène multivalente.

17. Dispositif de test analytique selon la revendication 15 ou la revendication 16, dans lequel la protéine de liaison d'antigène multivalente comprend une seule chaîne de polypeptides ayant les premier et second motifs de liaison de domaine reliés en série.

18. Dispositif de test analytique selon l'une quelconque de la revendication 15 ou de la revendication 17, dans lequel le réactif de liaison comprend une protéine de liaison d'antigène bivalente et le récepteur d'analyte est un anticorps ayant une spécificité de liaison à l'analyte.

19. Dispositif de test analytique selon l'une quelconque des revendications 15 à 18, dans lequel les seuls motifs de liaison de domaine sont des domaines variables de chaînes lourdes provenant d'une immunoglobuline naturel-lement dépourvue de chaînes légères.

20. Dispositif de test analytique selon l'une quelconque des revendications 15 à 19, dans lequel les seuls motifs de liaison de domaine sont des domaines variables de chaînes lourdes provenant d'une immunoglobuline Camelid.

21. Dispositif de test analytique selon l'une quelconque des revendications 15 à 20, dans lequel le réactif de liaison est un réactif de liaison marqué.

22. Dispositif de test analytique selon la revendication 21, dans lequel le marqueur est une particule de latex colorée ou un composé fluorescent.
